# EUROPEAN PATENT APPLICATION

(11) **EP 1 849 444 A1**
(43) Date of publication of application: **31.10.2007**
(21) Application number: 07106518.9
(22) Date of filing: 19.04.2007
(51) Int. Cl.: A61F 9/007

(54) **System for driving an ultrasonic handpiece with a class D amplifier**

(30) Priority: 21.04.2006 US 408724
(71) Applicant: Alcon, Inc., 6331 Hünenberg (CH)
(72) Inventor: Salehi, Ahmad, Irvine, CA 92614 (US); Nagarkar, Ajay, Oceanside, CA 92056 (US)
(74) Representative: Hanna, Peter William Derek

(57) **Abstract**

System for controlling an ultrasonic handpiece of an ocular surgical system, such as a phacoemulsification system. First and second signal sources generate first and second drive signals. The first signal is at a first frequency and is used to drive a cutting tip of the handpiece with a first type of motion. The second signal is at a second frequency and is used to drive the cutting tip with a second type of motion. The different motions can be generated with different first and second frequencies. The first and second signals can be summed or combined and provided to a class D amplifier, the output of which includes multiple frequency components or multiple signals of different frequencies to drive the cutting tip in different directions at the same time, for example, with simultaneous longitudinal and torsional motions.

## Description

### FIELD OF THE INVENTION

The present invention relates generally to the field of ophthalmic surgery and, more particularly, to a system and method for controlling different types of motion of a cutting tip of an ultrasonic handpiece using a class D amplifier.

### BACKGROUND

The human eye functions to provide vision by transmitting light through a clear outer portion called the cornea, and focusing the image by way of a lens onto a retina. The quality of the focused image depends on many factors including the size and shape of the eye, and the transparency of the cornea and lens. When age or disease causes the lens to become less transparent, vision deteriorates because of the diminished light that can be transmitted to the retina. This deficiency is medically known as a cataract. An accepted treatment for cataracts is to surgically remove the cataract and replace the lens with an artificial intraocular lens (IOL). In the United States, most cataractous lenses are removed using a surgical technique called phacoemulsification. During this procedure, a thin cutting tip or needle is inserted into the diseased lens and vibrated ultrasonically. The vibrating cutting tip liquefies or emulsifies the lens, which is aspirated out of the eye. The diseased lens, once removed, is replaced by an IOL.

A typical ultrasonic surgical device suitable for an ophthalmic procedure includes an ultrasonically driven handpiece, an attached cutting tip, an irrigating sleeve or other suitable irrigation device, and an electronic control console. The handpiece assembly is attached to the control console by an electric cable or connector and flexible tubings. A surgeon controls the amount of ultrasonic energy that is delivered to the cutting tip and applied to tissue by pressing a foot pedal. Tubings supply irrigation fluid to and draw aspiration fluid from the eye through the handpiece assembly.

The operative part of the handpiece is a centrally located, hollow resonating bar or horn that is attached to piezoelectric crystals. The crystals are controlled by the console and supply ultrasonic vibrations that drive both the horn and the attached cutting tip during phacoemulsification. The crystal / horn assembly is suspended within the hollow body or shell of the handpiece by flexible mountings. The handpiece body terminates in a reduced diameter portion or nosecone at the body's distal end. The nosecone is externally threaded to accept the irrigation sleeve. Likewise, the horn bore is internally threaded at its distal end to receive the external threads of the cutting tip. The irrigation sleeve also has an internally threaded bore that is screwed onto the external threads of the nosecone. The cutting tip is adjusted so that the tip projects only a predetermined amount past the open end of the irrigating sleeve.

A reduced pressure or vacuum source in the console draws or aspirates emulsified tissue from the eye through the open end of the cutting tip, horn bores and the aspiration line, and into a collection device. Aspiration of emulsified tissue is aided by a saline solution or other irrigant that is injected into the surgical site through the small annular gap between the inside surface of the irrigating sleeve and the cutting tip.

One known technique is to make the incision into the anterior chamber of the eye as small as possible in order to reduce the risk of induced astigmatism. The ends of the cutting tip and the irrigating sleeve are inserted into a small incision in the cornea, sclera, or other location. These small incisions result in very tight wounds that squeeze the irrigating sleeve tightly against the vibrating tip. Friction between the irrigating sleeve and the vibrating tip generates heat. The risk of the tip overheating and burning tissue is reduced by the cooling effect of aspirated fluid flowing inside the tip. One known cutting tip is ultrasonically vibrated along its longitudinal axis within the irrigating sleeve by the crystal-driven horn, thereby emulsifying the selected tissue in situ. Other known cutting tips use piezoelectric elements that can produce a combination of longitudinal and torsional motion. However, known devices and associated longitudinal and/or torsional motion of a cutting tip can be improved.

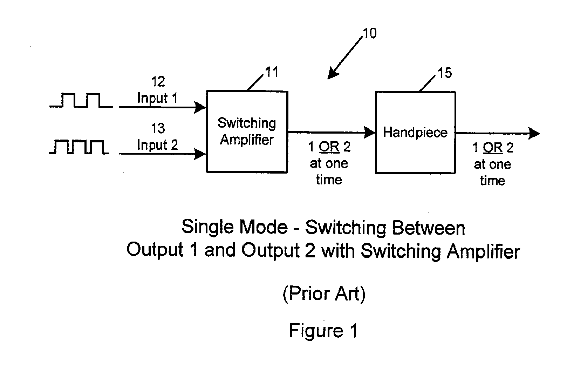

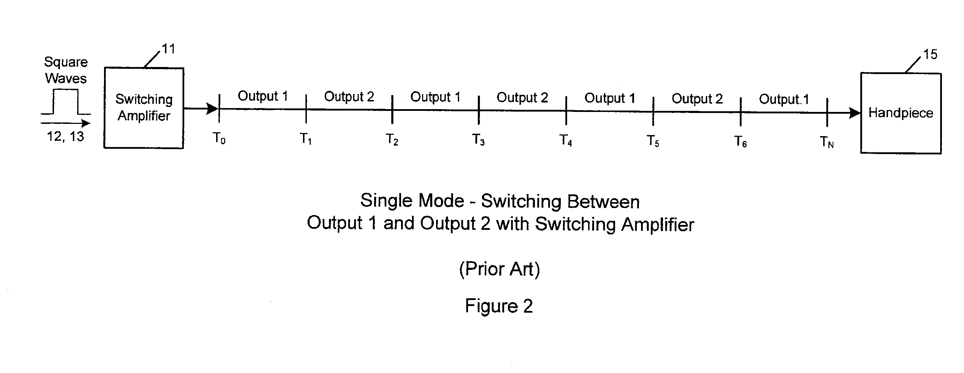

Referring to Figure 1, for example, known cutting tips are typically driven by switching amplifiers, which switch between different signals and different corresponding types of motion. Figure 1 generally illustrates a known system 10 that uses a switching amplifier 11, to alternately drive the cutting tip at different frequencies or with different types of motion at different times. The switching amplifier 11 receives a first input 12 and a second input 13. Given the design of a typical switching amplifier 11, both of the inputs 12 and 13 are typically square waves, which provide the necessary digital high and digital low signals to drive transistors in the switching amplifier 11. The switching amplifier 11 generates an output 14 that corresponds to either the first input 12 or the second input 13, as indicated by "1 OR 2" in Figure 1. In other words, the cutting tip of the handpiece 15 is either moved longitudinally or torsionally but not both longitudinally and torsionally simultaneously, as shown in Figure 2. These switching systems are generally referred to as "single-mode" systems since the cutting tip moves with one type of motion at a given time.

Known single-mode systems are not desirable for a number of reasons. First, they are not able to treat patients with different types of cutting tip motion simultaneously, which is generally referred to as "multi-mode" operation. Multi-mode treatments are desirable because, for example, torsional motion can achieve similar cutting results while generating less heat due to torsional motion being at lower frequencies than longitudinal motion. Further, known switching amplifiers are typically very inefficient and may have efficiency ratings of only 50% or lower. Known switching amplifiers can also generate substantial heat, which requires that handpieces and components thereof be designed in a particular manner to dissipate the heat, thus limiting handpiece designs. Known switching systems also consume substantial power, which is even more problematic at higher frequencies since components, such as capacitors, draw more current (and dissipate more heat) at higher frequencies. Known switching systems also include components that are relatively large in size, thus limiting designs and making the handpiece less user friendly.

Other systems provide for a combination of longitudinal and torsional movement, but they can also be improved. For example, U.S. Patent No. 5,722,945 describes a handpiece that includes an ultrasonic vibrator and a rotational motor. The motor is coupled to the vibrator which, is coupled to an aspirating tube to impart a combined rotary and longitudinal ultrasonic reciprocating motion to the tube, which moves a tip. These known systems, however, are not desirable since they require a motor and the associated motor coupling components, separate from the ultrasonic vibrator, to generate rotational motion. For example, these types of motor driven systems may require O-ring or other seals or couplings that can fail, as well as the motors themselves. The motor components increase the complexity, size and weight of the handpiece, and make the handpiece more difficult to control.

A need, therefore, exists for systems and methods for driving cutting tips of ultrasonic handpieces in various modes and that are more efficient, generate less heat, consume less power and allow for more flexible handpiece designs. Embodiments of the invention fulfill these unmet needs.

### SUMMARY

In accordance with one embodiment of the invention, a system for controlling an ultrasonic handpiece of a phacoemulsification surgical system includes first and second signal sources and a class D amplifier. The first signal source generates a first signal at a first frequency, and the second signal source generates a second signal at a second frequency. The first signal controls a first motion of a cutting tip of the ultrasonic handpiece, and the second signal controls a second motion of the cutting tip. The signals are inputs to the class D amplifier, which generates an amplified output having multiple frequency components that are used to move the cutting tip with different types of motion at the same time.

In accordance with yet another alternative embodiment, a system for controlling an ultrasonic handpiece of a phacoemulsification surgical system includes first and second signal sources and a class D amplifier, and a first signal generated by the first signal source controls longitudinal motion of a handpiece cutting tip, and a second signal generated by the second signal source controls torsional motion of the cutting tip. The first and second signals are provided as inputs to the class D amplifier, which generates an amplified output having multiple frequency components that move the cutting tip with different types of motion at the same time.

In another alternative embodiment, a system for controlling an ultrasonic handpiece of an ocular surgical system includes a first sinusoidal signal source that generates a first sinusoidal signal at a frequency of about 40 kHz to about 45 kHz and a second sinusoidal signal source that generates a second sinusoidal signal at a second frequency of about 30 kHz to about 34 kHz. The first signal controls longitudinal movement of a cutting tip of the handpiece, and the second signal controls torsional movement of the cutting tip. The system includes a class D amplifier, which receives as inputs the first and second signals and generates an output. The output has multiple frequency components that move the tip with longitudinal motion and torsional motion at the same time.

In various system embodiments, input signals, such as sinusoidal signals, are provided as inputs to a class D amplifier, which outputs a signal that controls movement of the cutting tip. For example, the tip can move with longitudinal and torsional motion at the same time without switching between amplified first and second signals. The signals sources can be oscillators that generate sinusoidal signals, and a summation element, such as a summing amplifier, can combine tow input signals into a third signal that includes multiple frequency components and that is provided as an input to the class D amplifier.

In various embodiments, the cutting tip can move in different directions under control of the class D amplifier output, e.g., with simultaneous torsional and longitudinal motion. Different types of motion can be achieved using signals at different frequencies, e.g., longitudinal movement can be controlled by a signal at about 40 kHz to about 45 kHz, and torsional movement can be controlled by a signal at about 30 kHz to about 34 kHz. With different types of motion, the cutting tip can move in different planes.

The ultrasonic handpiece includes a piezoelectric element and a horn coupled thereto. Exciting the piezoelectric element causes the horn to vibrate, thereby generating a first signal that drives the cutting tip of the handpiece. According to one embodiment, this causes the cutting tip to move longitudinally. The piezoelectric element can also be excited to cause the horn to vibrate, causing the cutting tip to move torsionally. According to one embodiment, torsional movement is generated as a result of apertures defined in the horn, resulting in longitudinal motion being converted into torsional motion.

### BRIEF DESCRIPTION OF THE DRAWINGS

Referring now to the drawings, in which like reference numbers represent corresponding parts throughout, and in which:
Figure 1 is a block diagram of a known single-mode system including a switching amplifier to drive a cutting tip in one direction at a time;
Figure 2 illustrates timing of the signals output by the switching amplifier shown in Figure 1;
Figure 3 generally illustrates an exemplary ophthalmic surgical system in which embodiments of the invention can be implemented;
Figure 4 is block diagram further illustrating components of an exemplary surgical system that can be used with embodiments of the invention;
Figure 5A generally illustrates an exemplary ultrasonic handpiece that can be used with embodiments of the invention;
Figure 5B further illustrates portions of an exemplary ultrasonic handpiece;
Figure 5C illustrates portions of Figure 5B in further detail;
Figure 6 is a flow chart illustrating a method for single mode operation of an ultrasonic handpiece using a class D amplifier according to one embodiment of the invention;
Figure 7 is a block diagram of a system that includes a class D class amplifier for single mode operation of an ultrasonic handpiece according to one embodiment of the invention;
Figure 8 illustrates timing of signals output by the class D amplifier shown in Figure 7;
Figure 9 is a flow chart illustrating a method for multi-mode operation of an ultrasonic handpiece using a class D amplifier according to an alternative embodiment of the invention;
Figure 10 is a block diagram of a system that includes a class D amplifier for multi-mode operation of an ultrasonic handpiece according to one embodiment of the invention;
Figure 11 is a block diagram of a system that includes a summing amplifier and a class D amplifier for multi-mode operation of an ultrasonic handpiece according to another embodiment of the invention;
Figure 12 illustrates timing of signals output by the class D amplifier shown in Figures 10 and 11;
Figure 13 is a flow chart illustrating a method for driving an ultrasonic handpiece with combined longitudinal and torsional motion using the handpiece shown in Figure 5;
Figure 14 is a perspective view of a piezoelectric crystal of an ultrasonic handpiece that can be driven by a class D amplifier according to an alternative embodiment;
Figure 15 is a flow chart illustrating a method for driving an ultrasonic handpiece with combined longitudinal and torsional motion using a handpiece having a crystal shown in Figure 14;
Figure 16A is a block diagram of an exemplary class D amplifier that can be used to drive an ultrasonic handpiece according to various embodiments;

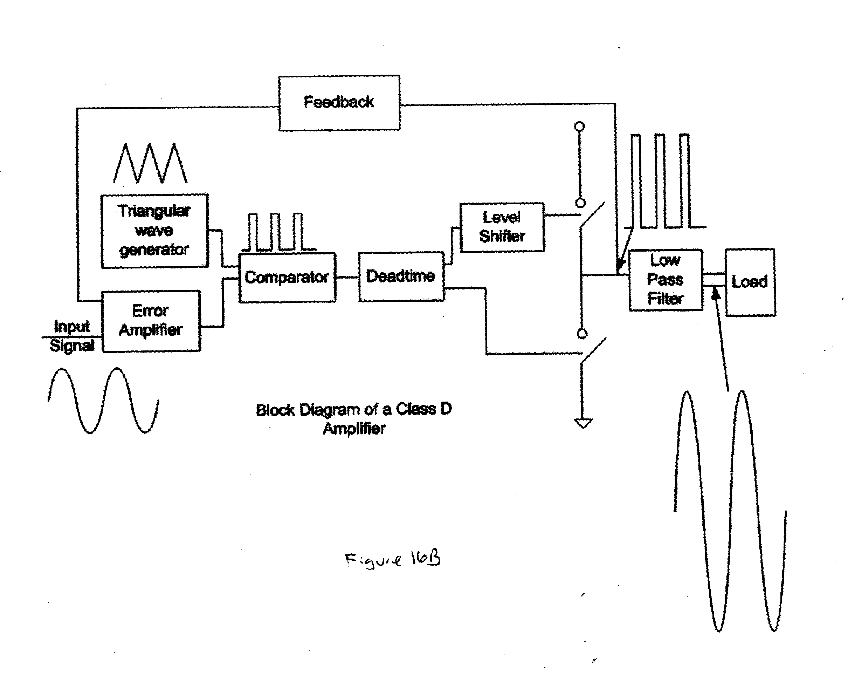

Figure 16B is a more detailed diagram of the class D amplifier shown in Figure 17A; and
Figure 16C illustrates signals at each stage of the class D amplifier shown in Figures 17A and 16B.

### DETAILED DESCRIPTION OF ILLUSTRATED EMBODIMENTS

Embodiments of the invention drive an ultrasonic handpiece using a class D amplifier for use in both single-mode operation, in which one drive signal is provided to the handpiece at a time, and in multi-mode operation, in which the cutting tip moves with both longitudinal and torsional or rotational motion. Embodiments advantageously eliminate the need for switching amplifiers, which are commonly used in known systems. Embodiments also advantageously eliminate the need for separate motors and related components to generate rotational motion since embodiments configure and control piezoelectric element and horn components of the handpiece to generate both longitudinal and torsional motion without the need for a separate motor. Embodiments overcome the shortcomings of known systems by using a class D amplifier or other amplifier with similar capabilities, such as a class T amplifier. Class D amplifiers are commonly used in audio applications, but the inventors have discovered that incorporating class D amplifiers into ultrasonic handpieces for use in ophthalmic surgery significantly improves handpiece operation, whether switching between drive signals, or when moving the cutting tip with both longitudinal and torsional motion. Embodiments provide these capabilities together with further benefits of increasing handpiece efficiency and reducing heat generation and power consumption, which allow more flexible and user friendly handpiece designs.

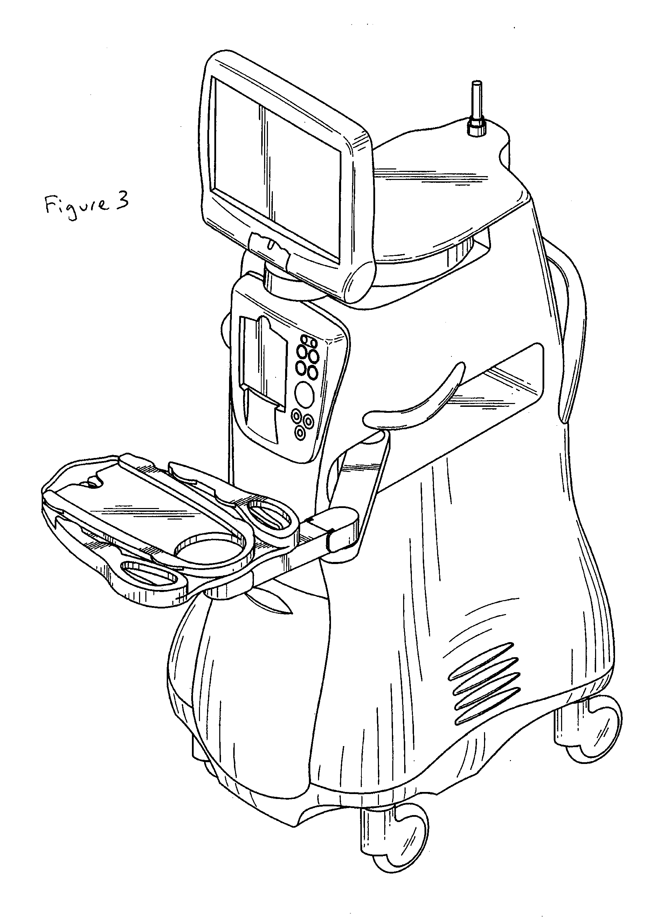

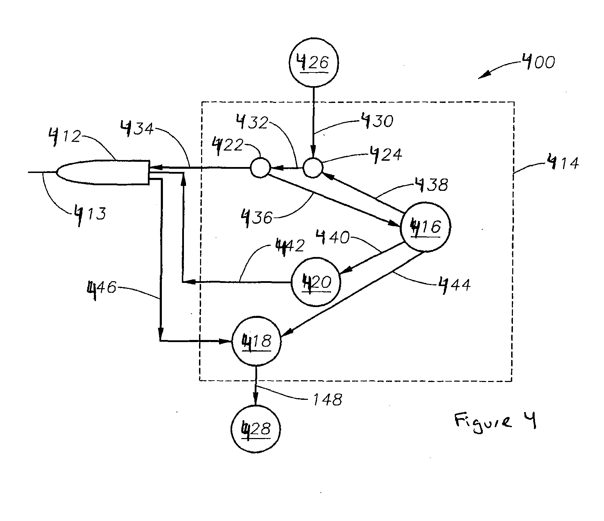

Figures 3-5C illustrate exemplary ocular surgical systems, in particular, phacoemulsification surgical systems, in which embodiments can be used. Figure 3 illustrates one suitable phacoemulsification surgical system that can be used with embodiments of the invention and represents the INFINITI^{®} Vision System available from Alcon Laboratories, Inc., 6201 South Freeway, Q-148, Fort Worth, Texas 76134. Persons skilled in the art will appreciate that embodiments can be implemented in other ultrasonic surgical systems, including those based on or related to the INFINITI^{®} system including, but not limited to, the LAUREATE^{™} system, also available from Alcon Laboratories, Inc.

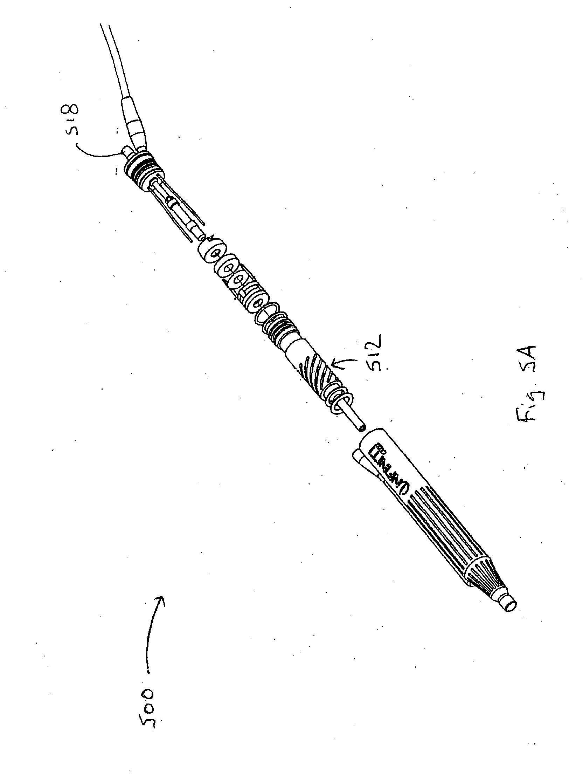

Referring to Figure 4, one suitable system 400 that is used to operate an ultrasound handpiece 412 includes a control console 414, which has a control module or CPU 416, an aspiration, vacuum or peristaltic pump 418, a handpiece power supply 420, an irrigation flow or pressure sensor 422 and a valve 424. The console 414 may be any commercially available surgical control console.

The CPU 416 may be any suitable microprocessor, micro-controller, computer or digital logic controller. The pump 418 may be a peristaltic, a diaphragm, or a Venturi pump. The power supply 420 may be any suitable ultrasonic driver, such as incorporated in the INFINITI^{®} and LAUREATE^{™} surgical systems. The irrigation pressure sensor 422 may be various commercially available sensors. The valve 424 may be any suitable valve such as a solenoid-activated pinch valve. An infusion of an irrigation fluid, such as saline, may be provided by a saline source 426, which may be any commercially available irrigation solution provided in bottles or bags.

In use, the irrigation pressure sensor 422 is connected to the handpiece 412 and the infusion fluid source 426 through irrigation lines 430, 432 and 434. The irrigation pressure sensor 422 measures the flow or pressure of irrigation fluid from the source 426 to the handpiece 412 and supplies this information to the CPU 416 through the cable 436. The irrigation fluid flow data may be used by the CPU 416 to control the operating parameters of the console 414 using software commands. For example, the CPU 416 may, through a cable 440, vary the output of the power supply 420 being sent to the handpiece 412 and the tip 413 though a power cable 442. The CPU 416 may also use data supplied by the irrigation pressure sensor 422 to vary the operation of the pump 418 and/or valves through a cable 444. The pump 418 aspirates fluid from the handpiece 412 through a line 446 and into a collection container 428 through line 448. The CPU 416 may also use data supplied by the irrigation pressure sensor 422 and the applied output of power supply 420 to provide audible tones to the user. Additional aspects of exemplary surgical systems can be found in U.S. Patent No. 6,261,283 (Morgan, et al.), the contents of which are incorporated herein by reference.

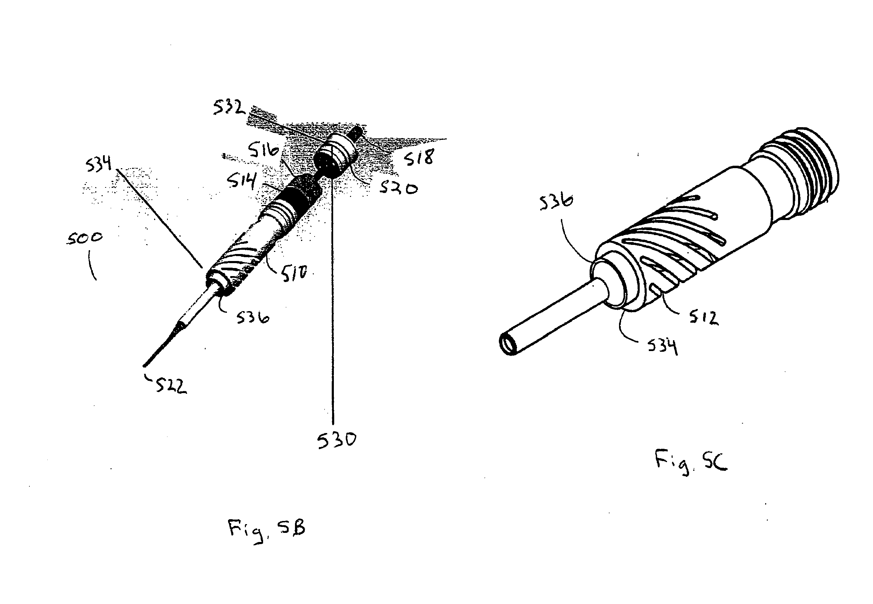

Referring to Figures 4 and 5A-C, various ultrasound handpieces 412 and cutting tips can be utilized. Exemplary handpieces 412 that can be used with embodiments of the invention include the Ozil^{™} and Ozil8^{™} ultrasonic handpieces, which are also available from Alcon Laboratories, Inc. Referring to Figure 5A, As best seen in FIG. 1 handpiece 500 of the present invention generally comprises ultrasonic horn 510, typically made from a titanium alloy. Horn 510 has a plurality of helical slits 512. A plurality (typically 1 or 2 pairs) of ring-shaped piezoelectric elements 514 are held by compression nut 516 against the horn 510. An aspiration tube or shaft 518 extends down the length of handpiece 500 through the horn 520, piezoelectric elements 514, the nut 516 and through a plug 520 at the distal end of handpiece 500. The aspiration tube 518 allows material to be aspirated through a hollow tip 522, which is attached to the horn 510, and through and out handpiece 500. The plug 520 seals the outer shell of handpiece 500 fluid tight, allowing the handpiece 500 to be autoclaved without adversely affecting piezoelectric elements 514. Additional grooves for sealing O-ring gaskets can be provided on the horn 520.

Referring to Figure 5C, in particular, the horn 510 contains a plurality of spiral slits 512. Preferably, the width of slits 512 is between 2% and 65% of the outside diameter of horn 510. This, of course, will affect how many slits 512 can be made on horn 510 (*e.g.,* if slits 24 are 65% of the diameter of horn, then only one slit may be cut into horn). The width of slits 512 can depend upon the desired about of torsional movement. The depth of slits 512 is preferably between about 4% and 45% of the outside diameter of horn 510. The slits 512 can have a flat or square cut bottom. Alternatively, the slits 512 can have a rounded or radiused bottom. The length of slits 512 is preferably between about 8% and 75% of the length of the larger diameter of horn 510. The pitch of slits 512 is preferably between about 125% and 500% of the larger diameter of horn 510. For example, a horn 510 having an outside diameter of 0.475" can have eight slits 512, having a width of 0.04", a depth of 0.140" (with a full radius bottom), a length of 0.7" and a pitch of 1.35". This configuration provides suitable torsional movement of horn 510 without compromising the longitudinal movement of horn 510.

The location of longitudinal and torsional nodal points (the points with zero velocity of the respective mode) is important for proper functioning of the handpiece 500. The torsional node 530 preferably is located at the proximal longitudinal node 532, so that the torsional node 530 and the longitudinal node 532 are coincident, e.g., both of which are located on the plug 520. The handpiece 500 also has a distal longitudinal node 534 located at reduced diameter portion 536 of the horn 510. Further aspects of a suitable handpiece 500 are provided in Patent Application Publication No. US 2006/0041220 A1, the contents of which are incorporated herein by reference.

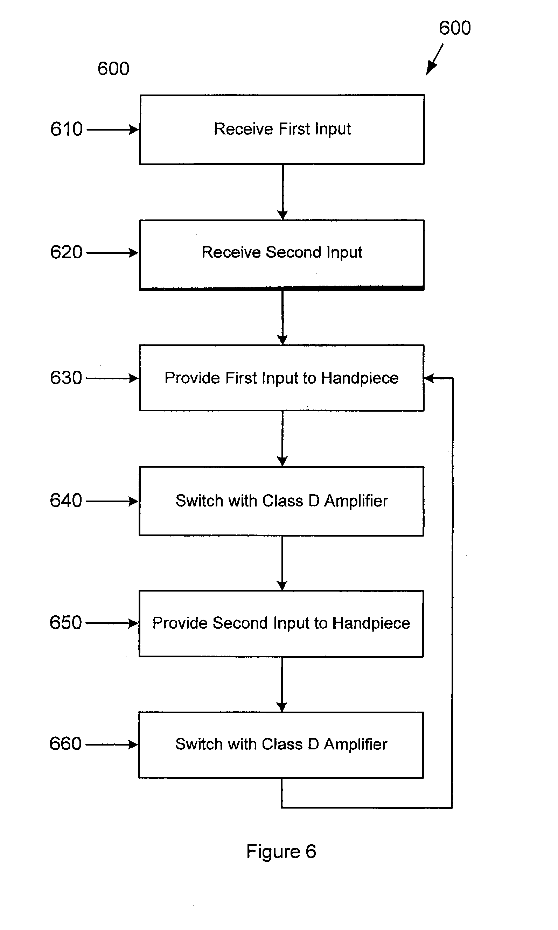

Referring to Figure 6, one embodiment is a method 600 for driving an ultrasonic handpiece (such as the handpiece 500 shown in Figures 5A-C) in single-mode operation by switching between different drive signal using a class D amplifier. In step 610, a first input or drive signal is received, e.g., as an input to the class D amplifier. In step 620, a second input or drive signal is received. In step 630, the class D amplifier outputs a first amplified signal that drives the ultrasonic handpiece. In step 640, after the first signal is active for a certain time, the class D amplifier switches from the first output to a second output so that in step 650, the second amplified signal drives the ultrasonic handpiece. After the second signal is active for a certain time, the class D amplifier switches from the second output back to the first output in step 660. The first output of the class D amplifier then drives the handpiece, and steps 630-660 are repeated as necessary.

Persons skilled in the art will appreciate that these method steps can be performed in various orders. For example, steps 610 and 620 may occur sequentially, in a different order or simultaneously. Further, persons skilled in the art will appreciate that a class D amplifier can be used to switch between two signals or, alternatively to switch among three or more signals depending on the class D amplifier capabilities.

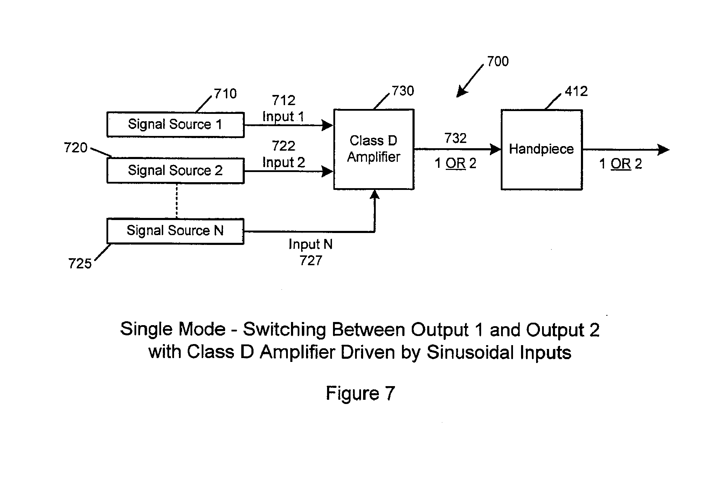

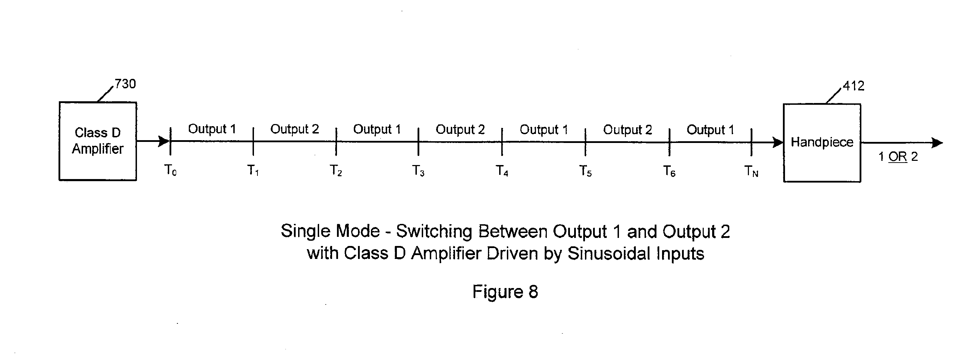

Figures 7 and 8 illustrate a system 700 for switching between different drive signals using a class D amplifier for driving an ultrasonic handpiece (such as the handpiece 500 shown in Figures 5A-C). According to one embodiment, the system 700 includes a first signal source 710, a second signal source 720 and a class D amplifier 730. Embodiments can be implemented using a class D amplifier, an amplifier derived from a class D amplifier or an amplifier having the same capabilities thereof. For example, a class T amplifier can be utilized. This specification refers to class D amplifiers for purposes of explanation and illustration, but "class D amplifier" is defined to include class T amplifiers and other related amplifiers having similar capabilities.

Two signal sources 710 (Signal Source 1) and 720 (Signal Source 2) (generally 710) are shown in Figure 7. Persons skilled in the art will appreciate that embodiments can be used for switching among various numbers of signal sources 725, identified as Signal Source N. For purposes of explanation and illustration, this specification refers to two signal sources. In the illustrated embodiment, the signal sources are oscillators or other sources that generate a first sinusoidal drive signal or input, Input 712, and a second sinusoidal drive signal or input, Input 722 (generally 712). The terms "drive signal" and "input" are used in this specification as including a signal used to power an ultrasonic handpiece, a signal used to tune or calibrate a handpiece, and a combination of such power and tuning or calibration signals. Drive signals 712 and 722 are provided to the class D amplifier 730, which switches between signals 712 and 722 so that only one of these drive signals is provided to the handpiece 412 at a given time, as shown in Figure 8.

Embodiments using a class D amplifier for single-mode operation provide a number of improvements over known systems that use switching amplifiers. For example, the system 700 operates with improved efficiency, which can be about 90% rather than about 50%. The system 700 also generates less heat relative to known systems, thus providing more flexibility in terms of component and system design, size, weight and heat dissipation. The system 700 also consumes less power than known systems, and these power advantages are particularly notable at higher frequencies.

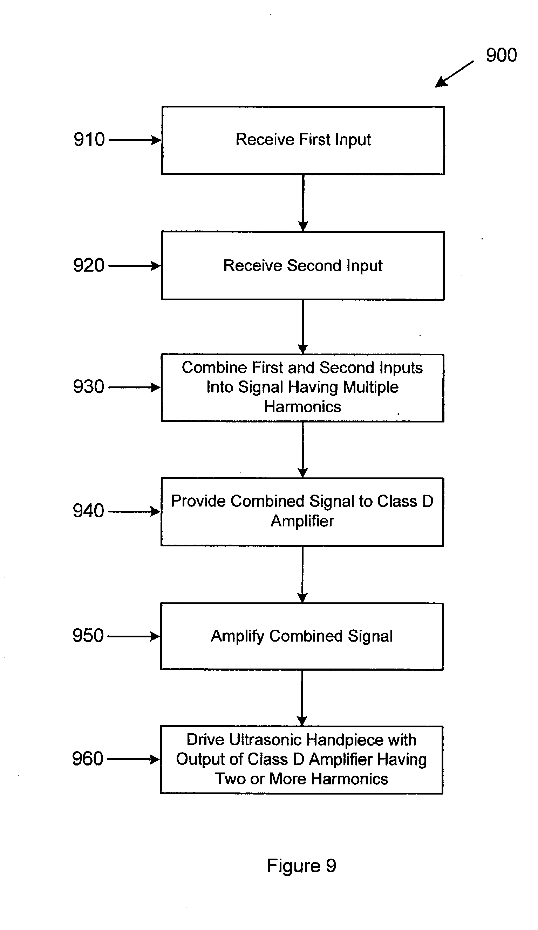

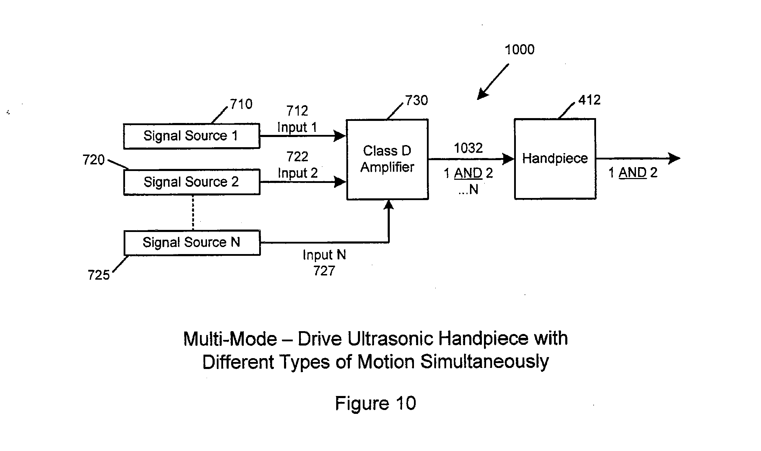

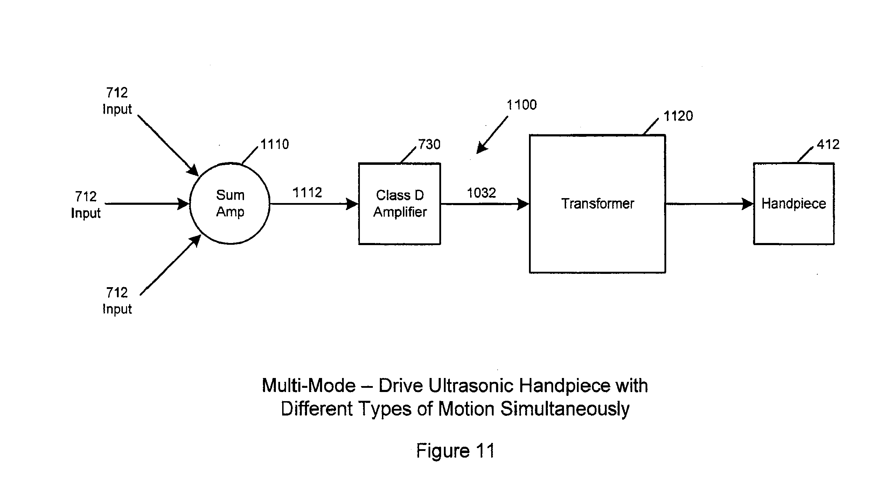

Referring to Figure 9, another embodiment of the invention is a method 900 for driving an ultrasonic handpiece (such as the handpiece shown in Figures 5A-C) in multi-mode operation by providing multiple drive signals from a class D amplifier to move a cutting tip of the handpiece in multiple directions at the same time. In step 910, a first input or drive signal is received, and in step 920, a second input or drive signal is received. In step 930 the inputs are combined using, for example, a summing amplifier, and the output of the summing amplifier is provided to a class D amplifier in step 940. In step 950, the combined signal is amplified, and the output of the class D amplifier is used to drive the handpiece in step 960. The signal provided by the class D amplifier to the handpiece includes multiple harmonics. Thus, the cutting tip of the handpiece moves in different directions or with different types of motion at the same time. Persons skilled in the art will appreciate that certain steps shown in Figure 9 can be omitted or performed in a different order. For example, it is not necessary to combine the signals in step 930. Rather, individual signals can be provided to a class D amplifier without using a summing amplifier, as shown in Figures 10 and 11.

Figure 10 illustrate a system 1000 for driving an ultrasonic handpiece (such as the handpiece 500 shown in Figures 5A-C) with different types of motion at the same time. Drive signals 712 are provided to the class D amplifier 730, which generates an output 1032. The output 1032 includes multiple harmonics or frequency components, in contrast to the output 732 (Figure 7), which has only one harmonic or frequency. Thus, the handpiece is driven with different signals, and the cutting tip moves with different types of motion at the same time.

In the embodiment illustrated in Figure 10, the drive signals 712 are provided to the amplifier 730 individually. However, in an alternative embodiment, shown in Figure 11, first and second drive signals 712 can be added together or combined by a summation unit 1110, which generates an output that is a third or combination signal 1112, which is fed to the class D amplifier 730.

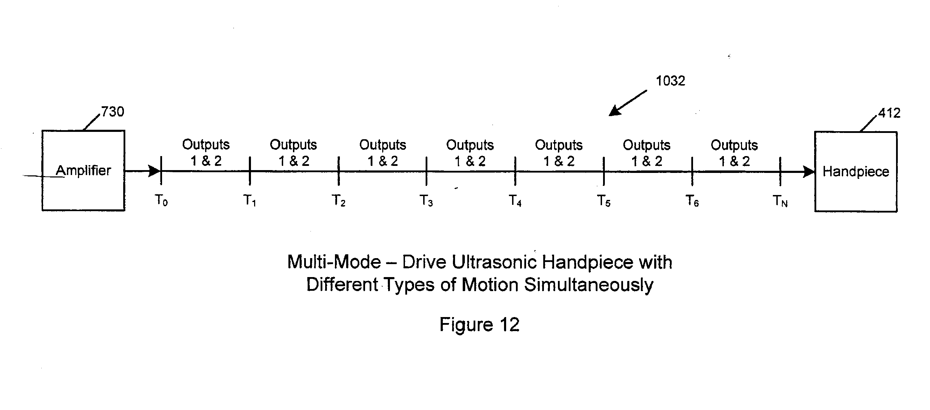

In the embodiment shown in Figure 11, the output 1112 of the summing component 1110 is a combination of the input signals. The output 1112 is typically at voltage levels between about 0 and 5 volts. The output 1112 is a signal with two or more frequency components or harmonics and is provided to the class D amplifier 730, which generates an output 1032. The output 1032 includes multiple frequency components or harmonics corresponding to the inputs 712, as shown in Figure 12.

Figure 11 also illustrates the output 1032 of the class D amplifier 730 being provided to a transformer 1120. The transformer 1120 is used to adjust the voltage level of the output 1032 of the class D amplifier 730 to a level that is suitable for the handpiece 412. For example, the output 1032 may be at a voltage level between about 0 and 30 volts. The transformer 1120 steps up the 0-30 volt level to a level of about 0-270 volts or another voltage that is suitable to drive the handpiece 412. The transformer 1120 also isolates or insulates other circuit components from the handpiece 412. Current and voltage feedbacks can be provided to ensure that the proper voltage and current are provided to the handpiece 412. The handpiece 412 moves with different types of motion at the same time under control of the output 1022 from the transformer 1120, as shown in Figure 12. Persons skilled in the art will appreciate that the voltage levels in the circuit can be adjusted as necessary. Further, the particular voltage levels described above are provided for purposes of explanation, not limitation, since different devices that can be used in embodiments may operate at different voltages.

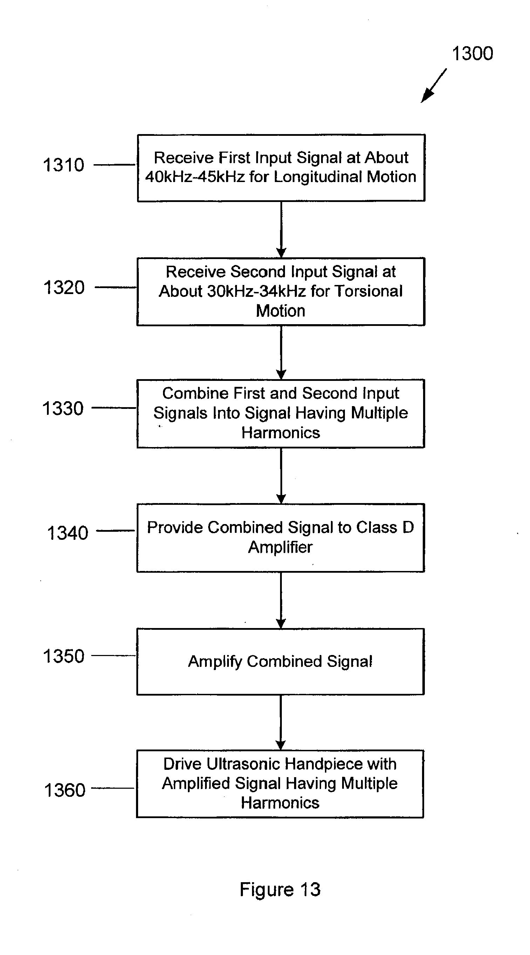

Referring to Figure 13, one embodiment of the invention is directed to a method 1300 for driving an ultrasonic handpiece, such as the handpiece 500 shown in Figure 5A-C and described in PCT Application No. PCT/US97/15952, using a class D amplifier to create both longitudinal vibratory motion and longitudinal motion. Longitudinal vibratory motion in the horn 510 is generated when piezoelectric crystals are excited. The slits 512 convert longitudinal motion of the crystals to torsional or oscillatory motion of the distal end of the horn 510.

According to one embodiment, in step 1310, a first input signal is received as an input to a class D amplifier. The first signal has a frequency between about 30kHz and 34 kHz and is used for torsional motion. In step 1320, a second signal is received, and the second signal can have a frequency of about 40 KHz and 45 KHz. The second signal is used for longitudinal motion. In step 1330, the first and second signals can be combined (if necessary), and in step 1340, the combined signal is provided to the class D amplifier. In step 1350, the class D amplifier amplifies the combined signal, and in step 1350, the output of the class D amplifier drives the cutting tip of the handpiece 500 so that the handpiece tip moves with combined longitudinal and torsional motion at the same time. As discussed above with respect to Figures 10 and 11, the first and second drive signals can be combined or provided directly to a class D amplifier.

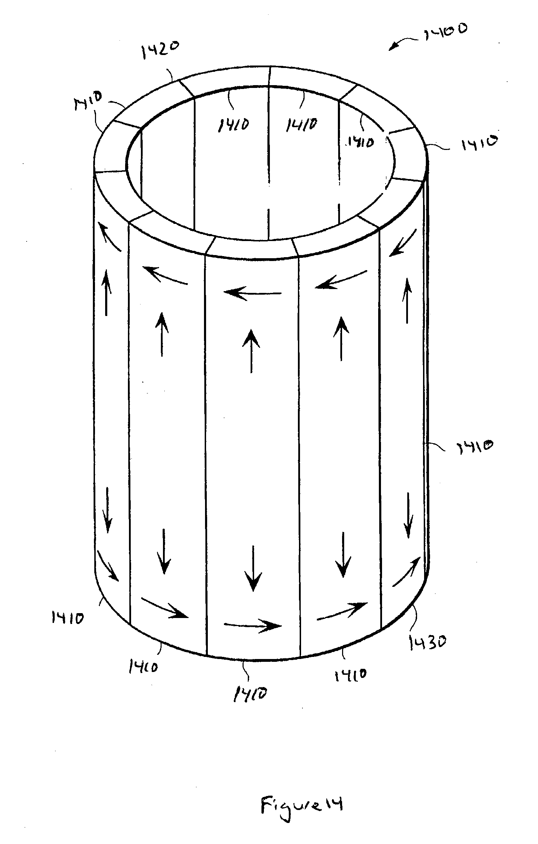

Figure 14 illustrates an exemplary crystal 1400 that can be used in a handpiece to supply ultrasonic vibrations that drive both the horn and the attached cutting tip during phacoemulsification. The exemplary crystal 1400 is a generally ring shaped crystal resembling a hollow cylinder and constructed from a plurality of crystal segments 1410 can generate signals having different frequencies to generate simultaneous longitudinal and torsional motion. Upper portions 1420 of segments 1410 may be polarized to produce clockwise motion while lower portions 1430 of segments 1410 may be polarized to produce counterclockwise motion or vice versa. The polarization of segments 1410 cause the crystal 1400 to twist when excited. In addition, the twisting motion of crystal 1400 will produce longitudinal motion, but such longitudinal motion will resonate at a different resonant frequency than the torsional motion.

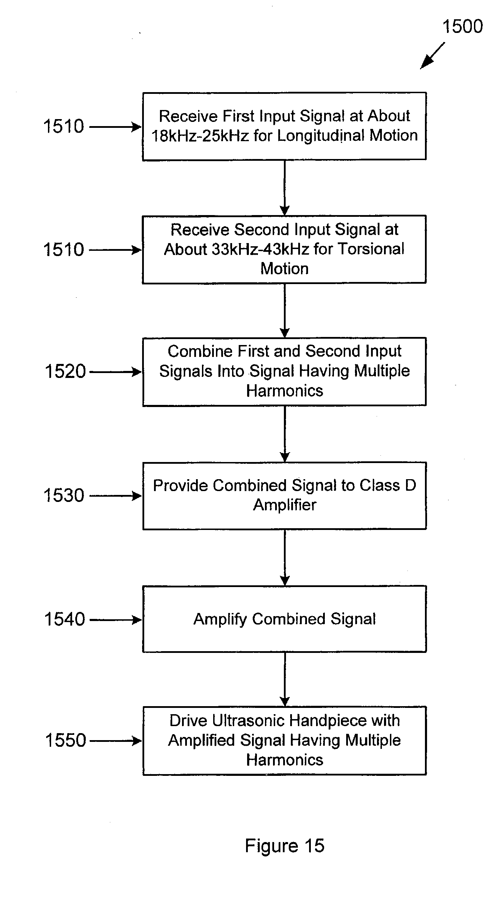

Referring to Figure 15, a method 1500 for driving an ultrasonic handpiece, such as the handpiece having a crystal 1500 described in U.S. Patent No. 6,402,769 to Boukhny, using a class D amplifier to create both longitudinal vibratory motion and longitudinal motion includes receiving a first input signal in step 1510, e.g., as an input to a class D amplifier. The first signal has a frequency between about 18 kHz and 25 kHz and is used for torsional motion. In step 1520, a second signal is received, and the second signal can have a frequency of about 33 KHz and 43 KHz and is used for longitudinal motion. In step 1530, the first and second signals can be combined (if necessary), and in step 1540, the combined signal is provided to the class D amplifier. In step 1550, the class D amplifier amplifies the combined signal, and in step 1550, the output of the class D amplifier drives the cutting tip of the handpiece with combined longitudinal and torsional motion at the same time. As discussed above with respect to Figure 10 and 11, the first and second drive signals can be combined or provided directly to an amplifier.

Thus, different types of motion of the cutting tip of the handpiece can define different planes of motion. A first type of motion can define a first plane, and a second, different type of motion can define a second plane. The two planes can be substantially perpendicular to each other when the first motion is longitudinal motion and the second motion is torsional motion. Other types of crystal designs, horn configurations and harmonics may result in planes of motion that are defined or arranged in other angular arrangements that may or may not be perpendicular.

Persons skilled in the art will recognize that different frequencies may be used depending upon the construction of piezoelectric crystals and the handpiece. Thus, the exemplary frequencies and frequency ranges for torsional and longitudinal motion are provided for purposes of explanation, not limitation. Further, various crystal and handpiece configurations can be used with the same or different frequencies to provide simultaneous longitudinal and torsional motion when driven by a class D amplifier.

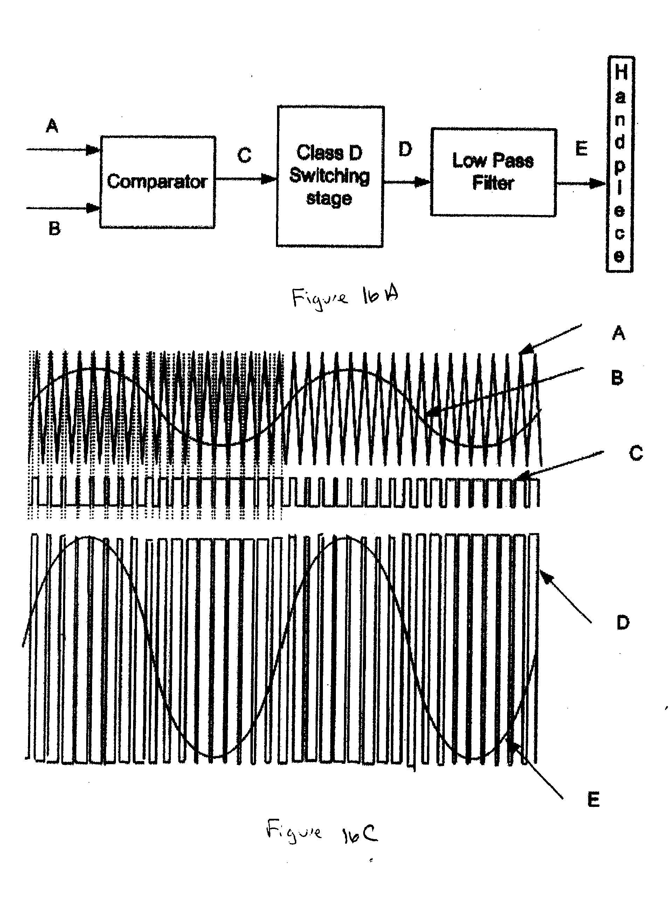

Class D amplifiers suitable for embodiments of the invention are well known and used in audio applications. Various known class D amplifiers can be incorporated into ophthalmic surgical systems to drive ultrasonic handpieces according to embodiments of the invention, including class D amplifier described in "Class D Amplifier for a Power Piezoelectric Load," by K. Agbossou et al. and Application Note AN-1071, "Class D Amplifier Basics," by J. Honda et al., International Rectifier, 233 Kansas Street, El Segundo, California, the contents of which are incorporated herein by reference. For reference, Figures 16A-C illustrate the components and operation of a typical class D amplifier. As illustrated, class D amplifiers generally operate by providing an input signal and a high frequency triangular wave to an error amplifier. The error amplifier generates a pulse width modulated (PWM) signal, which is provided to a controller. The controller drives Output/Power (O/P) switches, which are either on or off, thereby reducing power losses and increasing efficiency. A low pass filter reconstructs the original signal and removes a high frequency PWM carrier frequency.

Persons skilled in the art will appreciate that other amplifiers, such as class T amplifiers, can be used with embodiments of the invention. Embodiments advantageously use a class D amplifier or other suitable amplifier for driving a cutting tip to move with different types of motion at the same time rather than driving a cutting tip at one frequency at a time, while improving the operating parameters of the system. Embodiments provide a system that is more efficient, generates less heat, and dissipates substantially constant power over different frequencies. Further, embodiments provide a system that has smaller dimensions and less weight. Moreover, since less heat is generated, air-flow and power system requirements are relaxed. Thus, embodiments of the invention provide significant improvements over known ultrasonic handpieces and control systems that are less efficient, switch between different frequencies, generate more heat and use larger and additional components, such as switching amplifiers and separate motors for generating rotational motion.

Although references have been made in the foregoing description to various embodiments, persons of skilled in the art will recognize that insubstantial modifications, alterations, and substitutions can be made to the described embodiments without departing from the scope of embodiments.

## Claims

1. A system for controlling an ultrasonic handpiece of a phacoemulsification surgical system, comprising:
a first signal source that generates a first signal at a first frequency to control a first motion of a cutting tip of the ultrasonic handpiece;
a second signal source that generates a second signal at a second frequency to control a second motion of the cutting tip; and
a class D amplifier, wherein the class D amplifier receives the first and second signals as inputs and generates an amplified output having multiple frequency components that are used to move the cutting tip with different types of motion at the same time.

2. The system of claim 1, wherein the first and second signal sources are oscillators that generate sinusoidal signals that are provided to the class D amplifier.

3. The system of claim 1, further comprising a summation element, wherein the summation element receives the first and second signals and generates a third signal, the third signal being provided to the class D amplifier and being a combination of the first and second signals and having multiple frequency components.

4. The system of claim 3, wherein the summation element is a summing amplifier.

5. The system of claim 1, wherein the first signal controls longitudinal movement of the cutting tip, and the second drive signal controls torsional motion of the cutting tip.

6. The system of claim 5, wherein the first signal that controls longitudinal movement is at a frequency of about 40 kHz to about 45 kHz.

7. The system of claim 5, wherein the second signal that controls torsional movement is at a frequency of about 30 kHz to about 34 kHz.

8. The system of claim 1, wherein the first signal controls a first motion of the cutting tip, the first motion defining a first plane, the second signal controls a second motion of the cutting tip, the second motion defining a second plane, the first and second motions are different from each other, and the first and second planes are different from each other.

9. The system of claim 8, wherein the first and second planes are substantially perpendicular to each other.

10. The system of claim 1, wherein the class D amplifier generates an amplified output that moves the cutting tip with different types of motion at the same time without switching between amplified first and second signals.

11. The system of claim 1, wherein the output of the class D amplifier is a combination of amplified first and second drive signals.

12. The system of claim 1, wherein the first and second signals are first and second sinusoidal signals.

13. The system of claim 1, the ultrasonic handpiece including a piezoelectric element and a horn coupled to the piezoelectric element, wherein when the piezoelectric element is excited and causes the horn to vibrate, thereby generating the first signal to drive the cutting tip of the handpiece.

14. The system of claim 13, wherein the first signal drives the cutting tip to move longitudinally.

15. The system of claim 14, wherein the frequency of the first signal is about 40 kHz to about 45 kHz.

16. The system of claim 1, the ultrasonic handpiece including a piezoelectric element and a horn coupled to the piezoelectric element, wherein when the piezoelectric element is excited and causes the horn to vibrate, thereby generating the second signal to drive the cutting tip of the handpiece.

17. The system of claim 16, wherein the second signal causes the cutting tip to move torsionally.

18. The system of claim 17, wherein the frequency of the first signal is about 30 kHz to about 34 kHz.

19. The system of claim 17, wherein the horn defines a plurality of apertures, and the second signal is generated by the piezoelectric element causing the horn with the aperture to vibrate, thereby converting longitudinal motion into torsional motion.

20. The system of claim 1, the ultrasonic handpiece including a piezoelectric element and a horn coupled to the piezoelectric element, the horn defining a plurality of apertures, and exciting the piezoelectric element causing the horn to vibrate and generate the first signal at a frequency of about 40 kHz to about 45 kHz that causes the cutting tip to move longitudinally and the second signal at a frequency of about 30 kHz to about 34 kHz that causes the cutting tip to move torsionally.

21. The system of claim 20, wherein longitudinal motion is caused only be the excited piezoelectric element.

22. The system of claim 20, wherein the torsional motion is caused by a combination of the piezoelectric element and the horn, wherein torsional motion is caused the horn converting .

23. The system of claim 20, torsional motion being generated without a motor.

24. A system for controlling an ultrasonic handpiece of a phacoemulsification surgical system, comprising:
a first signal source that generates a first signal at a first frequency, the first signal controlling longitudinal motion of a cutting tip of the ultrasonic handpiece;
a second signal source that generates a second signal at a second frequency, the second signal controlling torsional motion of the cutting tip; and
a class D amplifier, wherein the class D amplifier receives the first and second signals as inputs and generates an amplified output having multiple frequency components that are used to move the cutting tip with different types of motion at the same time.

25. The system of claim 24, wherein the first and second signals are combined as a third signal, the third signal being provided as an input to the class D amplifier.

26. The system of claim 24, the first signal having a frequency of about 40 kHz to about 45 kHz.

27. The system of claim 24, the second signal having a frequency of about 30-34 kHz.

28. The system of claim 24, wherein the class D amplifier generates an amplified output that moves the cutting tip with longitudinal and torsional motion at the same time without switching between amplified first and second signals.

29. A system for controlling an ultrasonic handpiece of an ocular surgical system, comprising:
a first sinusoidal signal source that generates a first sinusoidal signal at a frequency of about 40 kHz to about 45 kHz and that controls longitudinal movement of a cutting tip of the handpiece;
a second sinusoidal signal source that generates a second sinusoidal signal at a second frequency of about 30 kHz to about 34 kHz and that controls torsional movement of the cutting tip;
a class D amplifier, wherein the class D amplifier receives the first and second sinusoidal signals and generates an output having multiple frequency components that move the cutting tip with longitudinal motion and torsional motion at the same time.

30. The system of claim 29, wherein the class D amplifier generates an amplified output that moves the cutting tip with longitudinal and torsional motion at the same time without switching between amplified first and second signals.

31. A system for controlling an ultrasonic handpiece of a phacoemulsification surgical system, comprising:
a first signal source that generates a first signal at a first frequency to control a first motion of a cutting tip of the ultrasonic handpiece;
a second signal source that generates a second signal at a second frequency to control a second motion of the cutting tip; and
a class D amplifier, wherein the class D amplifier receives the first and second signals as inputs and generates an output that switches between a first output at a first frequency and a second output at a second frequency to move the cutting tip in different directions at different times.
